# EUROPEAN PATENT APPLICATION

(11) **EP 1 557 430 A1**
(43) Date of publication of application: **27.07.2005**
(21) Application number: 03758800.1
(22) Date of filing: 23.10.2003
(51) Int. Cl.: C07K 16/18, C12N 15/09, C12N 5/10, C12P 21/08, A61K 39/395, A61P 9/00, A61P 9/02, A61P 9/04, A61P 9/06, A61P 9/12, A61P 13/10, A61P 13/12, A61P 25/16, A61P 25/18, A61P 25/20, A61P 25/22, A61P 25/24, A61P 25/28

(54) **ANTIBODY AND UTILIZATION OF THE SAME**

(30) Priority: 25.10.2002 JP 2002310364
(71) Applicant: Takeda Pharmaceutical Company Limited, Osaka 541-0045 (JP)
(72) Inventor: SHIMOMURA, Yukio, Tsukuba-shi, Ibaraki 300-4293 (JP); SUZUKI, Nobuhiro, Yodogawa-ku, Osaka-shi, Osaka 532-8686 (JP); MORI, Masaaki, Tsukuba-shi, Ibaraki 300-4293 (JP)
(74) Representative: Lewin, John Harvey
(86) International application number: PCT/JP2003/013528
(87) International publication number: WO 2004/037863

(57) **Abstract**

The present invention relates to antibodies specifically reacting with partial peptides in the C-terminal region of polypeptides having amino acid sequences represented by SEQ ID NOS: 1 through 8 or derivatives thereof, a method of quantifying urotensin II using the antibodies, and pharmaceutical compositions comprising the antibodies (e.g., for central nerve diseases, mental disorders, circulatory diseases, heart diseases, renal diseases, urinary tract disorders, or the like).

SEQUENCE LISTING
<110> Takeda Pharmaceutical Company Limited

<120> Antibody and its use

<130> 3104WOOP

<150> JP2002-310364
<151> 2002-10-25

<160> 9

<210> 1
<211> 11
<212> PRT
<213> Homo sapiens

<400> 1
Glu Thr Pro Asp Cys Phe Trp Lys Tyr Cys Val
                5                   10

<210> 2
<211> 12
<212> PRT
<213> Sus scrofa

<400> 2
Gly Pro Thr Ser Glu Cys Phe Trp Lys Tyr Cys Val
                5                   10

<210> 3
<211> 12
<212> PRT
<213> Sus scrofa

<400> 3
Gly Pro Pro Ser Glu Cys Phe Trp Lys Tyr Cys Val
                5                   10

<210> 4
<211> 12
<212> PRT
<213> Bos taurus

<400> 4
Gly Pro Ser Ser Glu Cys Phe Trp Lys Tyr Cys Val
                5                   10

<210> 5
<211> 14
<212> PRT
<213> Rattus norvegicus

<220>
<221>
<222>
<223> Xaa shows pyroglutamic acid

<400> 5
Xaa His Gly Thr Ala Pro Glu Cys Phe Trp Lys Tyr Cys Ile
                5                   10

<210> 6
<211> 12
<212> PRT
<213> Gobius spp

<400> 6

Ala Gly Thr Ala Asp Cys Phe Trp Lys Tyr Cys Val
                5                   10

<210> 7
<211> 8
<212> PRT
<213> Homo sapiens

<400> 7
Ala Cys Phe Trp Lys Tyr Cys Val
                5

<210> 8
<211> 14
<212> PRT
<213> Mus musculus

<220>
<221>
<222>
<223> Xaa shows pyroglutamic acid

<400> 8
Xaa His Gly Ala Ala Pro Glu Cys Phe Trp Lys Tyr Cys Ile
                5                   10

<210> 9
<211> 6
<212> PRT
<213> Homo sapiens

<400> 9
Cys Phe Trp Lys Tyr Cys
                5

## Description

### TECHNICAL FIELD

The present invention relates to an antibody having a binding specificity to a partial peptide in the C-terminal region of a polypeptide having the amino acid sequence represented by SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8, or a derivative of the polypeptide. More particularly, the present invention relates to an antibody, which is useful for developing a method of quantifying the aforesaid polypeptide or derivatives thereof, based on an antigen-antibody reaction, for development of diagnostic agents and preventive/therapeutic agents for diseases associated with the polypeptide or derivatives thereof; etc.

### BACKGROUND ART

It is known that various endogenous physiologically active peptides such as angiotensin II, bradykinin, endothelin, etc. relatet a regulation of cardiovascular systems such as cardiac function, blood pressure, etc. in mammals including human. In addition to these peptides, urotensin II was newly found to associate with cardiovascular systems recently, and has drawn attention as a new peptide of cardiovascular systems. Urotensin II is a peptide originally found in fish urophysis and is known to participate in cardiovascular regulation, osmotic regulation, lipid metabolism, etc. On the other hand, fish urotensin II was found to have hypotensive actions on mammals such as rat, etc. by intravenous administration or vasoconstrictor or vasodilatation actions on vessel specimens and the specific binding to labeled urotensin II was confirmed in the membrane preparations from rat vessels. It was thus predicted that the homolog of urotensin II would be present also in mammals to function as an endogenous peptide and its specific receptor would be present (J. Exp. Zool., 275, 226-238, 1996). And predictably, it was shown that the precursor gene of urotensin II was present in fish and a frog and further in mammals including mouse, rat and human (Proc. Natl. Acad. Sci. USA, 95, 15803-15808, 1998, FEBS Lett., 457, 28-32, 1999, WO 01/04298). Furthermore, urotensin II as a mature peptide processed from the precursor gene was purified and isolated from porcine spinal cords, indicating that urotensin II was actually present as a peptide also in mammals (Biochem. Biophys. Res. Commun., 265, 123-129, 1999, WO 00/32627). It was further clarified that human and rat GPR14 (SENR) (Genomics, 29, 335-344, 1995, Bichem. Biophys. Res. Commun., 209, 752-759, 1995), which is an orphan receptor with unknown ligand, was a functional receptor for urotensin II, based on the reactivity found in GPR14 receptor protein-expressed animal cells added with urotensin II as a ligand candidate (WO 01/04298, Nature, 401, 282-286, 1999, Biochem. Biophys. Res. Commun., 266, 174-178, 1999, Nature Cell Biol., 1, 383-385, 1999), or by purifying urotensin II as a ligand active substance from animal tissue extracts using the reactivity of the receptor-expressed cells as an indicator (Biochem. Biophys. Res. Commun., 265, 123-129, 1999, WO 00/3262).

Prior to the discovery of homologous peptides in mammals and their receptors, it had already been found that urotensin II had an extremely potent vasoconstrictive action, using goby urotensin II and rat thoracic aorta (Am. J. Phys., 21, R361-R366, 1987, Eur. J. Pharmacol., 149, 61-66, 1988), and the activity was confirmed also by using human urotensin II (Nature, 401, 282-286, 1999). It was further demonstrated that by intravenous administration in monkeys, urotensin II caused systemic vasoconstriction to decrease blood flow and induced heart failure by coronary vasoconstriction (Nature, 401, 282-286, 1999). From the foregoing, it was predicted that urotensin II may be involved in onset of heart disease, etc. as a new peptide associated with cardiovascular systems. However, subsequent investigations using isolated human vessels indicated that urotensin II did not always induce marked vasoconstriction in human coronary vessels or small vessels and the behavior on the circulatory system in human was not very potent (Br. J. Pharmacol., 131, 441-446, 2000, Am. J. Physiol. Heart Circ. Physiol., 280, H925-H928, 2001, Circulation, 103, 1378-1381, 2001). In the experiments where urotensin II was administered to human, there are both reports urotensin II reduced forearm blood flow (Br. J. Pharmacol., 135, 25-27, 2002) and did not affect the blood flow (Cardiovasc. Res., 53, 341-347, 2002). Recently, it was reported that the expression of urotensin II and its receptor right ventricle was enhanced in rat which developed pulmonary hypertension and right ventricular hypertrophy under low-oxygen condition (Heart Vessels, 16, 64-68, 2002) and in human as well, the expression of urotensin II was enhanced in the myocardium of patients with congestive heart failure (Lancet, 359, 1990-1997, 2002). Moreover, since it is reported that urotensin II reportedly induced hypertrophy in cultured cardiomyocytes of rat (FEBS Lett., 508, 57-60, 2001), it is suggested the possibility that urotensin II might participate in development of cardiac hypertrophy to cause heart failure. In addition, it is reported that blood levels of urotensin III have been found to be elevated in patients with heart failure (Lancet, 360, 545-546, 2002). Furthermore, it was reported that blood or urinary concentrations of urotensin II were increased in patients with renal dysfunction, etc. (Lancet, 358, 810-811, 2001, J. Hypertension, 19, 2185-2190, 2001), suggesting that urotensin II might take part in renal functions. It is also reported that GPR14 coexisted with cholinergic neurons in the mesopontine tegmental area (Brain Res., 923, 120-127, 2001) and intracerebroventricular injection of urotensin II elicited an increase in behavioral responses or increased anxiety in tests using rats (Psychopharmacology, 155, 426-433, 2001, WO 02/14513), suggesting that some central actions would be involved.

Further investigations are required for the involvement of urotensin II in physiological effects or diseases and hence, it has been earnestly desired to develop an assay system for detecting/quantifying urotensin II in a simple manner with high sensitivity.

### DISCLOSURE OF INVENTION

The present inventors have made extensive investigations to solve the foregoing problems and as a result, produced a plurality of monoclonal antibodies capable of recognizing urotensin II and developed an excellent method of determining urotensin II by using the antibodies. Further investigations have been made to accomplish the present invention.

That is, the present invention provides the following features, and the like.
(1) An antibody reacting specifically reacting with a partial peptide in the C-terminal region of a polypeptide having the amino acid sequence represented by SEQ ID NO: 1, SEQ ID NO: 2, SEQ ID NO: 3, SEQ ID NO: 4, SEQ ID NO: 5, SEQ ID NO: 6 or SEQ ID NO: 8, or a derivative of said polypeptide.
(2) The antibody according to (1), which specifically reacts with a partial peptide in the C-terminal region of a polypeptide having the amino acid sequence represented by SEQ ID NO: 1, SEQ ID NO: 2, SEQ ID NO: 3, SEQ ID NO: 4, SEQ ID NO: 5 or SEQ ID NO: 6, or a derivative of said polypeptide.
(3) The antibody according to (1), which specifically reacts with a peptide having the amino acid sequence represented by SEQ ID NO: 9.
(4) The antibody according to (1), wherein the partial peptide in the C-terminal region is a peptide having (i) the 5-10 amino acid sequence in SEQ ID NO: 1, (ii) the 6-11 amino acid sequence in SEQ ID NO: 2, SEQ ID NO: 3, SEQ ID NO: 4 or SEQ ID NO: 6, (iii) the 8-13 amino acid sequence in SEQ ID NO: 5, (iv) the 2-7 amino acid sequence in SEQ ID NO: 7, or (v) the 8-13 amino acid sequence in SEQ ID NO: 8.
(5) The antibody according to (1), which is a monoclonal antibody.
(6) The antibody according to (1), which is labeled.
(7) The antibody according to (1), which is a neutralizing antibody.
(8) The antibody according to (7), which neutralizes the activity of a polypeptide having the amino acid sequence represented by SEQ ID NO: 1, SEQ ID NO: 2, SEQ ID NO: 4, SEQ ID NO: 5, SEQ ID NO: 6, SEQ ID NO: 7 or SEQ ID NO: 8, or a derivative thereof.
(9) The antibody according to (7), which neutralizes the activity of a polypeptide having the amino acid sequence represented by SEQ ID NO: 1, SEQ ID NO: 2, SEQ ID NO: 4, SEQ ID NO: 5 or SEQ ID NO: 6.
(10) The antibody according to (5), which is shown by AUII5-6-10a capable of being produced from a hybridoma cell shown by AUII5-6-10 (FERM BP-8221).
(11) The antibody according to (5), which is shown by AUII103-5-41a capable of being produced from a hybridoma cell shown by AUII103-5-41(FERM BP-8220).
(12) A hybridoma cell capable of producing the antibody according to (5).
(13) The hybridoma cell according to (12), which is shown by AUII103-5-41 (FERM BP-8220).
(14) The hybridoma cell according to (12), which is shown by AUII5-6-10 (FERM BP-8221).
(15) A method of producing the antibody according to (5), which comprises culturing the hybridoma cell according to (12) in vivo or in vitro and collecting the antibody according to (5) from the body fluid or its culture.
(16) A pharmaceutical comprising the antibody according to (1).
(17) The pharmaceutical according to (16), which is a preventive/therapeutic agent for central nerve diseases, mental disorders, circulatory diseases, heart diseases, renal diseases or urinary tract disorders.
(18) A diagnostic agent comprising the antibody according to (1).
(19) The diagnostic agent according to (18), which is a diagnostic agent for central nerve diseases, mental disorders, circulatory diseases, heart diseases, renal diseases or urinary tract disorders.
(19a) The diagnostic agent according to (18), which is a diagnostic agent for mental disorders.
(20) A method of quantifying a polypeptide having the amino acid sequence represented by SEQ ID NO: 1, SEQ ID NO: 2, SEQ ID NO: 3, SEQ ID NO: 4, SEQ ID NO: 5, SEQ ID NO: 6 or SEQ ID NO: 8, or a derivative thereof, which comprises using the antibody according to (1).
(21) A method of quantifying a polypeptide having the amino acid sequence represented by SEQ ID NO: 1, SEQ ID NO: 2, SEQ ID NO: 3, SEQ ID NO: 4, SEQ ID NO: 5, SEQ ID NO: 6 or SEQ ID NO: 8, or a derivative thereof, in a test fluid, which comprises competitively reacting the antibody according to (1), a test fluid and a labeled form of polypeptide having the amino acid sequence represented by SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8, or a derivative of said polypeptide, and determining a ratio of the labeled polypeptide having the amino acid sequence represented by SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8, or a derivative thereof, bound to the antibody.
(22) A method for diagnosis of a disease associated with a polypeptide having the amino acid sequence represented by SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8, or a derivative thereof, which comprises using the antibody according to (1).
(23) A method of preventing/treating central nerve diseases, mental disorders, circulatory diseases, heart diseases, renal diseases or urinary tract disorders, which comprises administering an effective dose of the antibody according to (1) to a mammal.
(24) Use of the antibody according to (1) for manufacturing a preventive/therapeutic agent for central nerve diseases, mental disorders, circulatory diseases, heart diseases, renal diseases or urinary tract disorders.

### BRIEF DESCRIPTION OF THE DRAWINGS

FIG. 1 shows the results of antibody titers of mice immunized with goby urotensin II, which were examined using biotinylated goby urotensin II and HRP-labeled avidin. In the figure, symbols □(open square), ■(closed square), ○(open circle), ●(closed circle), Δ(open triangle), ▲(closed triangle), ◇(open diamond) and ◆(closed diamond) designate mice No. 1, No. 2, No. 3, No. 4, No. 5, No. 6, No. 7 and No. 8, respectively.
FIG. 2 shows typical examples for screening of hybridomas after cell fusion using mice immunized with goby urotensin II. In the figure, symbols □(open square) and ■ (closed square) designate the results with addition of no porcine urotensin II-1 and the results with addition of porcine urotensin II-1.
FIG. 3 shows the results of reactivities of AUII5-6-10a with human urotensin II (-□-), porcine urotensin II-1(-○-), bovine urotensin II (-Δ-), rat urotensin II (-◇-) and goby urotensin II (-x-), which were examined by competitive EIA using biotinylated goby urotensin II and HRP-labeled avidin.
FIG. 4 shows the results of reactivities of AUII103-5-41a with human urotensin II (-□-), porcine urotensin II-1(-○-), bovine urotensin II (-Δ-), rat urotensin II (-◇-) and goby urotensin II (-x-), which were examined by competitive EIA using biotinylated goby urotensin II and HRP-labeled avidin.
FIG. 5 shows the results of neutralizing effects of UII5-6-10a on arachidonate metabolite releasing activities of human urotensin II (-□-), porcine urotensin II-1(-○-), bovine urotensin II (-Δ-), rat urotensin II (-◇-) and goby urotensin II (-x-) from rat GPR14 receptor expression CHO cells.
FIG. 6 shows the suppressing effects of AUII5-6-10a on anxiety-like behaviors by intraventricular administration. In the figure, A, B and C on the abscissa designate the mouse IgG group (non-stressed), the mouse IgG group (exposed to restraint stress) and the AUII5-6-10a group (exposed to restraint stress), respectively, and the ordinate designates the count of peeping behavior.

### BEST MODE FOR CARRYING OUT THE INVENTION

Throughout the specification, the proteins (polypeptides) are represented in accordance with the conventional way of describing peptides, that is, the N-terminus (amino terminus) at the left hand and the C-terminus (carboxyl terminus) at the right hand. In the proteins used in the present invention, including a polypeptide having the amino acid sequence represented by SEQ ID NO:1, the C-terminus may be in any form of a carboxyl group, a carboxylate, an amide and an ester.

The polypeptide having the amino acid sequence represented by SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8, and derivatives thereof are sometimes collectively referred to as the peptide of the present invention. In addition, the polypeptide having the amino acid sequence represented by SEQ ID NO: 9 and derivatives thereof are also included in the peptide of the present invention.

The derivatives described above include, for example, peptides wherein a part of amino acid residues in the amino acid sequence represented by SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8 are substituted with a substitutable group, a part of the amino acid residues is deleted, the amino acid residues, etc. are added/inserted, and the like.

Examples of the derivatives of polypeptide having the amino acid sequence represented by SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8 include (i) those wherein at least 1 or 2 (e.g., 1 to 5, preferably 1 or 2) amino acids in the amino acid sequence described above are deleted, (ii) those, to which at least 1 or 2 (e.g., 1 to 5, preferably 1 or 2) amino acids in the amino acid sequence described above are added, (iii) those wherein at least 1 or 2 (e.g., 1 to 5, preferably 1 or 2) amino acids in the amino acid sequence described above are inserted, or (iv) those wherein at least 1 or 2 (e.g., 1 to 5, preferably 1 or 2) amino acids in the amino acid sequence described above are substituted with other amino acids.

The derivatives described above further include those, wherein a part of amino acid residues in the polypeptide having the amino acid sequence represented by SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8 is substituted with substitutable group(s) (e.g., Cys, hydroxyl group, etc.), those wherein a part of the amino acid residues is deleted and a part of the amino acid residues is substituted with a substitutable group(s) (e.g., Cys, hydroxyl group, etc.), and the like.

As the partial peptide in the C-terminal region of the peptide of the present invention, there are, for example, (i) a peptide having the 5-10 amino acid sequence in SEQ ID NO: 1, (ii) a peptide having the 6-11 amino acid sequence in SEQ ID NO: 2, SEQ ID NO: 3, SEQ ID NO: 4 or SEQ ID NO: 6, (iii) a peptide having the 8-13 amino acid sequence in SEQ ID NO: 5, (iv) a peptide having the 2-7 amino acid sequence in SEQ ID NO: 7, (v) a peptide having the 8-13 amino acid sequence in SEQ ID NO: 8, (vi) peptides wherein a part of amino acid residues (e.g., one amino acid residue) in these peptides is substituted with a substitutable group, and the like.

As the partial peptide in the N-terminal region of the peptide of the present invention, there are, for example, (i) a peptide having the 1-5 amino acid sequence in SEQ ID NO: 1, (ii) a peptide having the 1-6 amino acid sequence in SEQ ID NO: 2, SEQ ID NO: 3, SEQ ID NO: 4 or SEQ ID NO: 6, (iii) a peptide having the 1-8 amino acid sequence in SEQ ID NO: 5, (iv) a peptide having the 1-8 amino acid sequence in SEQ ID NO: 8, (v) peptides wherein a part of amino acid residues (e.g., one amino acid residue) in these peptides is substituted with a substitutable group, and the like.

The antibodies specifically reacting with the partial peptides at the C terminus of the peptide of the present invention may be any antibodies, which are capable of specifically reacting with the partial peptides at the C-terminus of the peptide of the present invention, and include antibodies specifically reacting with:
(i) a peptide having the 5-10 or 4-11 amino acid sequence in SEQ ID NO: 1;
(ii) a peptide having the 6-11 or 5-12 amino acid sequence in SEQ ID NO: 2, SEQ ID NO: 3, SEQ ID NO: 4 or SEQ ID NO: 6;
(iii) a peptide having the 8-13 or 7-14 amino acid sequence in SEQ ID NO: 5;
(iv) a peptide having the 2-7 amino acid sequence in SEQ ID NO: 7;
(v) a peptide having the 8-13 or 7-14 amino acid sequence in SEQ ID NO: 8; and,
(vi) peptides wherein a part of amino acid residues (e.g., one amino acid residue) in these polypeptides is substituted with a substitutable group, and the like.

More preferably, the antibodies specifically reacting with the partial peptides in the C-terminal region in the peptide of the present invention are monoclonal antibodies. Preferred examples of the monoclonal antibodies are a monoclonal antibody shown by AUII5-6-10a capable of being produced from a hybridoma cell shown by AUII5-6-10(FERM BP-8221), a monoclonal antibody shown by AUII103-5-41a capable of being produced from a hybridoma cell shown by AUII103-5-41(FERM BP-8220), etc.

As such, the antibodies specifically reacting with the partial peptide in the C-terminal region in the peptide of the present invention are capable of reacting with the peptide of the present invention by recognizing a specific amino acid sequence at the C terminus of the peptide of the present invention.

The antibodies specifically reacting with the partial peptides in the N-terminal region of the peptide of the present invention may be any antibodies that are capable of specifically reacting with the partial peptides in the N-terminal region of the peptide of the present invention. Examples of such antibodies include antibodies specifically reacting with (i) a peptide having the 1-5 amino acid sequence in SEQ ID NO: 1, (ii) a peptide having the 1-6 amino acid sequence in SEQ ID NO: 2, SEQ ID NO: 3, SEQ ID NO: 4 or SEQ ID NO: 6, (iii) a peptide having the 1-8 amino acid sequence in SEQ ID NO: 5, (iv) a peptide having the 1-8 amino acid sequence in SEQ ID NO: 8, and (v) peptides wherein a part of amino acid residues (e.g., one) in these polypeptides is substituted with a substitutable group, and the like.

As the antibodies specifically reacting with the partial peptides in the N-terminal region of the peptide of the present invention, monoclonal antibodies are preferred. More preferred examples of such antibodies are antibodies which are capable of specifically reacting with the partial peptides in the N-terminal region of the peptide of the present invention but are not reactive any partial peptide in the C-terminal region.

As such, the antibodies specifically reacting with the partial peptides in the N-terminal region of the peptide of the present invention can be reacted with the peptide of the present invention by recognizing a specific amino acid sequence at the N terminus in the peptide of the present invention described above.

Preparation of antigens for the antibodies of the present invention and production of the antibodies are described below.

### (1) Preparation of antigen

The antigen used to produce the antibodies of the present invention includes, for example, the peptide of the present invention, synthetic peptides having one or at least two antigenic determinants which are the same as the antigenic determinant of the peptide of the present invention, etc. (which are hereinafter sometimes merely referred to as the antigen of the present invention).

The peptide of the present invention can be produced (a) from tissues or cells of mammals (e.g., human, bovine, rat, mouse, swine, monkey, etc.), fishes (e.g., goby, etc.) by known methods or modifications, (b) through chemical synthesis by known peptide synthesis using a peptide synthesizer, etc., or (c) by culturing a transformant having a DNA encoding the peptide of the present invention.
(a) Where the antigen of the present invention is prepared from tissues or cells of those mammals or fish, the antigen of the present invention can be prepared by homogenizing the tissues or cells, extracting the homogenate with an acid, an alcohol, etc., and applying the combination of salting-out, dialysis, gel filtration, chromatographies such as reversed phase chromatography, ion exchange chromatography, affinity chromatography, etc. to the resulting extract to perform purification/isolation.
(b) Examples of the synthetic peptide used in chemical synthesis of the antigen of the peptide of the present invention include peptides having the same structure as in the antigen of the present invention purified from naturally occurring peptides and peptides containing 1 or at least 2 amino acid sequences having the same as the amino acid sequence at an optional portion consisting of at least 2, preferably at least 3 amino acids in the amino acid sequence for the peptide of the present invention, etc.
(c) Where the peptide of the present invention is manufactured using a transformant containing a DNA, the DNA can be prepared by known cloning methods (those described in, e.g., Molecular Cloning (2nd ed.; J. Sambrook et al., Cold Spring Harbor Lab. Press, 1989), etc.). The cloning methods include (1) a method for obtaining a transformant containing a DNA encoding the peptide of the present invention by hybridization method from cDNA library using a DNA probe or DNA primer designed under the amino acid sequence of the peptide of the present invention, (2) a method for obtaining a transformant containing a DNA encoding the peptide of the present invention by PCR method using a DNA primer designed based on the amino acid sequence of the peptide of the present invention, and the like.

The peptide of the present invention as an antigen can be prepared (1) by known peptide synthesis method or (2) by cleaving a peptide having the amino acid sequence represented by SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8 with an appropriate peptidase.

The peptide synthesis may be any of, for example, solid phase synthesis method and liquid phase synthesis method. That is, the objective peptide can be produced by condensing the partial peptides or amino acids, which can construct the said peptide, with the remaining part of the peptide and, where the product contains protecting groups, removing these protecting groups. Known methods for condensation and elimination of the protecting groups are described in (i) or (ii) below.

### (i) M. Bodanszky and M. A. Ondetti, Peptide Synthesis, Interscience Publishers, New York (1966)

### (ii) Schroeder and Luebke, The Peptide, Academic Press, New York (1965)

After completion of the reaction, the peptide can be purified and isolated by a combination of conventional purification methods such as solvent extraction, distillation, column chromatography, liquid chromatography, recrystallization, etc. When the peptide obtained by the above methods is in a free form, the peptide can be converted into an appropriate salt by a known method; conversely when the peptide is obtained in a salt form, the salt can be converted into a free form by a known method.

The amide form of the peptide can be prepared using commercially available resins that are suitable for amide formation. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids, in which α-amino groups and functional groups on the side chains are appropriately protected, are condensed on the resin in accordance with the sequence of the objective peptide according to known various condensation methods. At the end of the reaction, the peptide is excised from the resin, at the same time, the respective protecting groups are removed and the objective peptide is obtained. Alternatively, chlorotrityl resin, oxime resin, 4-hydroxybenzoic acid type resin, etc. are employed to take out the partially protected peptide and the protecting groups are removed in a conventional manner to give the objective peptide.

For condensation of the protected amino acids described above, a variety of activation reagent available for peptide synthesis can be used, and carbodiimides are preferably employed. Examples of such carbodiimides include DCC, N,N'-diisopropylcarbodiimide, N-ethyl-N'-(3-dimethylaminopropyl)carbodiimide, etc. For activation by these activation reagents, the protected amino acids in combination with a racemization inhibitor (e.g., HOBt, HOOBt, etc.) are added directly to the resin, or the protected amino acids are previously activated in the form of symmetric acid anhydrides, HOBt esters or HOOBt esters, followed by adding the thus activated protected amino acids to the resin. Solvents suitable for use to activate the protected amino acids or condense with the resin may be appropriately chosen from solvents that are known to be usable for peptide condensation reactions. Examples of such solvents, which are used for the activation of the protected amino acids or for the condensation with the resin, are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; tertiary amines such as pyridine, etc.; ethers such as dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to peptide binding reactions and is usually selected in the range of approximately -20°C to 50°C. The activated amino acid derivatives are used generally in an excess of 1.5 to 4 times. When the condensation is found to be insufficient for the peptide bond-forming reaction as a result of tests using the ninhydrin reaction, the condensation can be completed by repeating the condensation reaction without removal of the protecting groups. When the condensation is yet insufficient even after repeating the reaction, unreacted amino acids are acetylated with acetic anhydride or acetylimidazole to avoid any possible effect on the subsequent reaction.

Examples of the protecting groups used to protect the amino groups of the starting compounds include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc. Examples of the protecting groups of a carboxyl group include, in addition to a C₁₋₆ alkyl group, a C₃₋₈ cycloalkyl group and a C₇₋₁₄ aralkyl group, 2-adamantyl, 4-nitrobenzyl, 4-methoxybenzyl, 4-chlorobenzyl, phenacyl group and benzyloxycarbonyl hydrazide, t-butoxycarbonyl hydrazide, trityl hydrazide and the like.

The hydroxyl group of serine and threonine can be protected through, for example, its esterification or etherification. Examples of the groups appropriately used for the esterification include a lower (C₁₋₆) alkanoyl group, such as acetyl group, etc.; an aroyl group such as benzoyl group, etc., and a group derived from carbonic acid such as benzyloxycarbonyl group, ethoxycarbonyl group, etc. Examples of a group suitable for the etherification include benzyl group, tetrahydropyranyl group, t-butyl group, etc.

Examples of groups for protecting the phenolic hydroxyl group of tyrosine include Bzl, Cl-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, Bom, Bum, Boc, Trt, Fmoc, etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the activated amino acids, in which the amino groups are activated in the starting material, the corresponding phosphoric amides are employed.

As a method for eliminating (split off) the protecting groups, for example, catalytic reduction under hydrogen gas flow in the presence of a catalyst such as Pd-black, Pd-carbon, etc.; an acid treatment with anhydrous hydrofluoric acid, methanesulfonic acid, trifluoromethanesulfonic acid or trifluoroacetic acid, or a mixture solution of these acids; a treatment with a base such as diisopropylethylamine, triethylamine, piperidine, piperazine, etc.; and reduction with sodium in liquid ammonia; or the like. The elimination of the protecting groups by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group used as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of the functional groups that should not be involved in the reaction of the starting materials, protecting groups, elimination of the protecting groups and activation of the functional groups involved in the reaction may be appropriately selected from known groups and known means.

In another method for obtaining the amides of the peptide, for example, the α-carboxyl group of the carboxy terminal amino acid is first amidated; the peptide chain is then extended to a desired length toward the amino group side. Thereafter, a peptide in which only the protecting group of the N-terminal α-amino group in the peptide chain has been eliminated from the peptide and a peptide (or amino acids) in which only the protecting group of the C-terminal carboxyl group has been eliminated are prepared. The two peptides are condensed in a mixture of the solvents described above. The details of the condensation reaction are the same as described above. After the protected peptide obtained by the condensation is purified, all the protecting groups are eliminated by the method described above to be able to give the desired crude peptide. This crude peptide is purified by various known purification means. Lyophilization of the major fraction is able to give the amide of the desired peptide.

To prepare the esterified peptide, for example, the α-carboxyl group of the carboxy terminal amino acid is condensed with a desired alcohol to prepare the amino acid ester, which is followed by procedure similar to the preparation of the amidated peptide above to be able to give the ester form of the desired peptide.

The antigen of the present invention may be provided for direct immunization in its immobilized form. The antigen of the present invention may also be bound or adsorbed to an appropriate carrier and the complex produced may be provided for immunization. A mixing ratio of the carrier to the antigen of the present invention (hapten) may be in any ratio of any type, as long as the antibody can be efficiently produced to the antigen of the present invention. A high molecular carrier conventionally used to produce an antibody to a hapten may be used in a weight ratio of 0.1 to 100 based on 1 of hapten. As such a high molecular carrier, there are used a naturally occurring high molecular carrier and a synthetic high molecular carrier. Examples of the naturally occurring high molecular carrier used are serum albumin from mammals such as bovine, rabbit, human, etc., thyroglobulins from mammals such as bovine, rabbit, etc., hemoglobins from mammals such as bovine, rabbit, human, sheep, etc or KHL hemocyanin.

As the synthetic high molecular carrier, there may be used, for example, a variety of latexes including polymers or copolymers, etc., such as polyamino acids, polystyrenes, polyacryls, polyvinyls, polypropylenes, etc.

For coupling of the hapten and the carrier, a variety of condensing agents can be used. Examples of the condensing agents, which are advantageously employed, are diazonium compounds such as bis-diazotized benzidine through crosslinking of tyrosine, histidine or tryptophan; dialdehyde compounds such as glutaraldehyde, etc. through crosslinking of amino groups therebetween; diisocyanate compounds such as toluene-2,4-diisocyanate, etc.; dimaleimide compounds such as N,N'-o-phenylenedimaleimide, etc. by crosslinking of thiols therebetween; maleimide activated ester compounds by crosslinking of an amino group with a thiol group; carbodiimide compounds by crosslinking of an amino group with a carboxyl group; etc. In the crosslinking of amino groups with each other, one amino group is reacted with an activated ester reagent (e.g., SPDP, etc.) having dithiopyridyl and then reduced to introduce the thiol group, whereas another amino group is introduced with a maleimide group using a maleimide activated ester reagent, and the two groups may be reacted with each other.

### (2) Preparation of monoclonal antibody

The antigen of the present invention is administered to wann-blooded animal independently itself or together with carriers or diluents to the site where the production of antibody is possible by administration routes such as intraperitoneally, intravenously, subcutaneously, etc. In order to potentiate the antibody productivity upon the administration, complete Freund's adjuvants or incomplete Freund's adjuvants may be administered. The administration is usually carried out once in every 2 to 6 weeks and approximately 2 to 10 times in total. Examples of the warm-blooded animal are monkeys, rabbits, dogs, guinea pigs, mice, rats, sheep, goats, chicken, etc. with mice being preferred for the preparation of monoclonal antibodies.

In the preparation of monoclonal antibodies, from warm-blooded animals, e.g., mice, immunized with the antigen of the present invention, the animal wherein the antibody titer is noted is selected, then the spleen or lymph node is collected after 2 to 5 days from the final immunization and antibody-producing cells contained therein are fused with myeloma cells to give hybridomas capable of producing monoclonal antibodies to the peptide of the present invention. Measurement of the antibody titer of the peptide of the present invention in antisera may be made, for example, by reacting a labeled form of the peptide of the present invention, which will be described later, with the antiserum followed by assaying the binding activity of a marker bound to the antibody. The fusion may be operated, for example, by the known Kohler and Milstein method [Nature, 256, 495 (1975)]. Examples of fusion accelerators are polyethylene glycol (PEG), Sendai virus, etc., of which PEG is preferably employed. Examples of the myeloma cells are NS-1, P3U1, SP2/0, AP-1, etc. In particular, P3U1 or the like is preferably employed. A preferred ratio in count of the antibody-producing cells (spleen cells) to the myeloma cells used is within a range of approximately 1:1 to 20:1. When PEG (preferably, PEG 1000 to PEG 6000) is added in a concentration of approximately 10 to 80% followed by incubation generally at 20 to 40°C, preferably at 30 to 37°C generally for 1 to 10 minutes, an efficient cell fusion can be carried out.

Various methods can be used for screening hybridomas capable of producing the antibodies of the present invention. Examples of such methods include a method which comprises adding the hybridoma supernatant to a solid phase (e.g., microplate) adsorbed with the peptide of the present invention or its partial peptides directly or together with a carrier, then adding an anti-immunoglobulin antibody (when mouse cells are used for the cell fusion, anti-mouse immunoglobulin antibody is used) labeled with a radioactive substance, an enzyme or the like, or Protein A and detecting the monoclonal antibodies of the present invention bound to the solid phase; a method which comprises adding the hybridoma supernatant to a solid phase adsorbed with an anti-immunoglobulin antibody or Protein A, adding the peptide of the present invention labeled with a radioactive substance, an enzyme, etc. and detecting the monoclonal antibodies of the present invention bound to the solid phase; etc. Screening and plating of the monoclonal antibodies of the present invention can be performed generally in a medium for animal cells (e.g., RPMI 1640) containing 10-20% fetal calf serum and supplemented with HAT (hypoxanthine, aminopterin and thymidine). The antibody titer in the hybridomas culture supernatant can be assayed as in the assay for the antibody titer of the antibody of the present invention in the antisera described above.

Separation and purification of the monoclonal antibody to the peptide of the present invention can be carried out by methods applied to conventional separation and purification of immunoglobulins, as in the conventional methods for separation and purification of polyclonal antibodies (e.g., salting-out, alcohol precipitation, isoelectric point precipitation, electrophoresis, adsorption and desorption with ion exchangers (e.g., DEAE), ultracentrifugation, gel filtration, or a specific purification method which involves collecting only an antibody with an activated adsorbent such as an antigen-binding solid phase, Protein A, Protein G, etc. and dissociating the binding to obtain the antibody; and the like).

As described above, the antibody of the present invention can be produced by culturing hybridoma cells in a warm-blooded animal *in vivo* or *in vitro* and collecting the antibody of the present invention from the body fluids or culture.

The antibody of the present invention can sensitively quantify the peptide of the present invention.

Hereinafter, uses of the antibody of the present invention including the method of quantifying the peptide of the present invention (immunoassay), pharmaceuticals comprising the antibody of the present invention, etc. are described in detail.

### (1) Method of quantifying the peptide of the present invention

Using the antibody of the present invention, the peptide of the present invention can be assayed and also detected by tissue staining, or the like. For these purposes, the antibody molecule itself may be used, or F(ab')2, Fab' or Fab fractions of the antibody molecule may be used.

The quantification method using the antibody of the present invention is not particularly limited. Any quantification method can be used, so long as the amount of antibody, antigen or antibody-antigen complex corresponding to the amount of antigen (e.g., the amount of the peptide of the present invention) in a fluid to be tested can be detected by chemical or physical means and the amount of the antigen can be calculated from a standard curve prepared from standard solutions containing known amounts of the antigen.

For such an assay method, for example, the sandwich method, the competitive method, the immunometric method, nephrometry, etc. are used, and the competitive method described below are more preferred in terms of sensitivity and specificity.

### 1) Competitive method

The competitive method is the determination method for quantifying the peptide of the present invention in a test fluid by competitively reacting the antibody of the present invention, the test fluid and a labeled form of the peptide of the present invention, and measuring a ratio of the labeled form of the peptide of the present invention bound to the antibody.

Preferably, quantification of the peptide of the present invention in a test fluid by the competitive method is carried out using, e.g., solid phase technique.

Specifically, there are the following procedures using anti-mouse IgG antibody as an antibody for solid phase, which comprise:
(a) reacting (i) the antibody of the present invention (e.g., the monoclonal antibody shown byAUII5-6-10a or AUII103-5-41a, etc.), (ii) the peptide of the present invention, which is labeled with biotin and (iii) a test fluid; adding avidin labeled with HRP (horse radish peroxidase) to the plate; after the reaction, assaying the HRP activity adsorbed onto the solid phase to quantify the peptide of the present invention;
(b) adding (i) the antibody of the present invention (e.g., the monoclonal antibody shown by AUII5-6-10a or AUII103-5-41a, etc.), (ii) the peptide of the present invention, which is labeled with HRP and (iii) a test fluid; after the reaction, assaying the HRP activity adsorbed onto the solid phase to quantify the peptide of the present invention; etc.

### 2) Sandwich method

The sandwich method is a determination method for quantify the peptide of the present invention ina test fluid by reacting the antibody of the present invention immobilized on a carrier with a labeled form of the antibody of the present invention and a test fluid, and assaying the activity of a marker to quantify the peptide of the present invention in the test fluid.

Preferably, the sandwich method includes:
(i) A determination method of the peptide of the present invention in a test fluid, which comprises reacting the antibody specifically reacting with a partial peptide in the N-terminal region of the peptide of the present invention immobilized onto a carrier, a labeled form of the antibody (monoclonal antibody shown by AUII5-6-10a or AUII103-5-41a) specifically reacting with a partial peptide in the C-terminal region of the peptide of the present invention and the test fluid, and assaying the activity of a labeling agent;
(ii) A determination method of the peptide of the present invention in a test fluid, which comprises reacting the antibody (monoclonal antibody shown by AUII5-6-10a or AUII103-5-41a) specifically reacting with a partial peptide in the C-terminal region of the peptide of the present invention immobilized onto a carrier, a labeled form of the antibody specifically reacting with a partial peptide in the N-terminal region of the peptide of the present invention and the test fluid, and assaying the activity of a labeling agent; etc.

In the sandwich method, a test fluid is reacted with the immobilized antibody specifically reacting with a partial peptide in the C-terminal region of the peptide of the present invention, or the antibody specifically reacting with a partial peptide in the N-terminal region of the peptide of the present invention (primary reaction) and then the test fluid is reacted with a labeled antibody specifically reacting with a partial peptide in the C-terminal region of the peptide of the present invention, or a labeled antibody specifically reacting with a partial peptide in the N-terminal region of the peptide of the present invention (secondary reaction), and the activity of a labeling agent on the immobilizing carrier is assayed, whereby the amount of the peptide of the present invention in the test fluid can be quantified. The primary and secondary reactions may be performed simultaneously or at time intervals. The labeling agent and immobilizing methods may be based on those described above. Further, in immunoassay by the sandwich method, the antibodies used for solid phase or for labeling are not necessarily one species, but a mixture of two or more species of antibodies may be used for purposes of increasing the measurement sensitivity, etc. In the method of assaying the peptide of the present invention by the sandwich method, for example, when the antibodies used in the primary reaction recognize the partial peptides in the C-terminal region of the peptide of the present invention, the antibodies used in the secondary reaction are preferably those recognizing partial peptides other than the C-terminal region (i.e., the N-terminal region). When the antibodies used for the primary reaction recognize partial peptides in the N-terminal region of the peptide of the present invention, the antibodies used in the secondary reaction, antibodies recognizing partial peptides other than the N-terminal region (i.e., the C-terminal region) are preferably employed.

### 3) Immunometric method

In immunometric method, an antigen in a test fluid and an antigen immobilized to a solid phase are competitively reacted with a given amount of a labeled antibody of the present invention, followed by separating the solid phase from the liquid phase; or the antigen in a test fluid is reacted with an excess amount of a labeled antibody of the present invention, then an antigen immobilized to a solid phase is added to bind a unreacted, labeled antibody of the present invention to the solid phase, followed by separating the solid phase from the liquid phase. Next, the labeling amount in any of the phases is measured to determine the amount of the antigen in the test fluid.

### 4) Nephrometry

In nephrometry, the amount of insoluble sediment, which is produced as a result of the antigen-antibody reaction in a gel or in a solution, is measured. When the amount of an antigen in a test fluid is small and only a small amount of the sediment is obtained, laser nephrometry utilizing laser scattering can be suitably used.

In the quantification methods 1) through 4) described above, labeling agents used for the assay method using labeling substances are not particularly limited but radioisotopes, enzymes, fluorescent substances, luminescent substances, etc. are employed. Preferred examples of the radioisotopes include, but are not limited thereto, [¹²⁵I], [¹³¹I], [³H], [¹⁴C], etc. The enzymes described above are not particularly limited but are preferably enzymes which are stable and have a high specific activity, and include β-galactosidase, β-glucosidase, an alkaline phosphatase, a peroxidase, malate dehydrogenase, etc. The fluorescent substances described above are not particularly limited but examples include fluorescamine, fluorescein isothiocyanate, etc. The luminescent substances described above are not particularly limited but examples include luminol, a luminol derivative, luciferin, lucigenin, etc. Furthermore, the compounds of the biotin-avidin system may be used for binding of an antibody to a labeling agent.

For immobilization of antigen or antibody, physical adsorption may be used. Chemical binding techniques conventionally used for insolubilization or immobilization of proteins, enzymes, etc. may also be used. For carriers, there are used, e.g., insoluble polysaccharides such as agarose, dextran, cellulose, etc.; synthetic resin such as polystyrene, polyacrylamide, silicon, etc., and glass or the like.

In applying each of these immunoassays to the method of the present invention, it is not necessary to set any special condition, operation, etc. The assay system for the peptide of the present invention may be constructed in addition to the conditions or operations conventionally used for each of the methods, taking into account the technical consideration of one skilled in the art. For the details of such conventional technical means, reference may be made to a variety of reviews, reference books, etc. (for example, Hiroshi Irie (ed.): "Radioimmunoassay" (published by Kodansha, 1974); Hiroshi Irie (ed.): "Radioimmunoassay; Second Series" (published by Kodansha, 1979); Eiji Ishikawa, et al. (ed.): "Enzyme Immunoassay" (published by Igaku Shoin, 1978); Eiji Ishikawa, et al. (ed.): "Enzyme Immunoassay" (Second Edition) (published by Igaku Shoin, 1982); Eiji Ishikawa, et al. (ed.): "Enzyme Immunoassay" (Third Edition) (published by Igaku Shoin, 1987); "METHODS IN ENZYMOLOGY" Vol. 70 (Immunochemical Techniques (Part A)); ibid., Vol. 73 (Immunochemical Techniques (Part B)); ibid., Vol. 74 (Immunochemical Techniques (Part C)); ibid., Vol. 84 (Immunochemical Techniques (Part D: Selected Immunoassays)); ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies)) (all published by Academic Press); etc.). Thus, the antibody of the present invention enables to quantify the peptide of the present invention with high sensitivity and is useful for clarification of the physiological functions of the peptide of the present invention and for the prevention/treatment or diagnosis of diseases/symptoms associated with the peptide of the present invention.

The peptide of the present invention has effects including a vascular smooth muscle contractile effect, a myocardiotrophic action, an anxiety increasing action, etc.

By determining the amount of the peptide of the present invention contained in body fluids (blood, plasma, serum, urine, etc.) using the antibody of the present invention, it is possible to diagnose for diseases associated with the peptide of the present invention [for example, central nerve diseases (e.g., Alzheimer's disease, Parkinsonian syndrome, Pick's disease, Huntington's disease, senile dementia, cerebrovascular dementia, etc.), mental disorders (e.g., anxiety, depression, insomnia, schizophrenia, phobia, etc.), circulatory diseases (e.g., hypertension, hypotension, etc.), heart diseases (e.g., heart failure, arrhythmia, long QT syndrome, dilated congestive cardiomyopathy, hypertrophic cardiomyopathy, pulmonary hypertension, etc.), renal diseases (e.g., nephritis, renal failure, interstitial renal disorders, etc.), urinary tract disorders (e.g., pollakiuria, urinary incontinence, etc.), or the like] and so on. In addition, the antibody of the present invention can be used to detect the peptide of the present invention in test fluids such as body fluids, tissues, etc. Moreover, the antibody of the present invention is available for preparation of antibody columns used to purify the peptide of the present invention, detection of the peptide of the present invention in each fraction upon purification, analysis of the behavior of the peptide of the present invention in cells to be tested; etc.

### (2) Pharmaceutical comprising the antibody of the present invention

The antibody of the present invention has the effects of neutralizing the peptide of the present invention to inhibit the effects exhibited by the peptide of the present invention, such as the vascular smooth muscle contractile effect, myocardiotrophic action, anxiety increasing action, etc. Thus, the antibody of the present invention can be used as pharmaceuticals such as preventive/therapeutic agents or diagnostic agents, etc. for the diseases associated with the peptide of the present invention [for example, central nerve diseases (e.g., Alzheimer's disease, Parkinsonian syndrome, Pick's disease, Huntington's disease, senile dementia, cerebrovascular dementia, etc.), mental disorders (e.g., anxiety, depression, insomnia, schizophrenia, phobia, etc.), circulatory diseases (e.g., hypertension, hypotension, etc.), heart diseases (e.g., heart failure, arrhythmia, long QT syndrome, dilated congestive cardiomyopathy, hypertrophic cardiomyopathy, pulmonary hypertension, etc.), renal diseases (e.g., nephritis, renal failure, interstitial renal disorders, etc.), urinary tract disorders (e.g., pollakiuria, urinary incontinence, etc.), or the like].

The preventive/therapeutic agent comprising the antibody of the present invention is safe and low toxic, and can be administered parenterally or orally to human or mammals (e.g., rats, rabbits, sheep, swine, bovine, cats, dogs, monkeys, etc.) as a liquid preparations or as a pharmaceutical composition of appropriate dosage form.

The antibody of the present invention may be administered in its intact form or in the form of an appropriate pharmaceutical composition. The pharmaceutical composition used for administration may contain the antibody of the present invention or its salt, a pharmacologically acceptable carrier and a diluent or an excipient. Such a pharmaceutical composition is provided in a dosage form suitable for oral or parenteral administration.

Examples of the composition for parenteral administration are injectable preparations, suppositories, etc. The injectable preparations may include dosage forms such as intravenous, subcutaneous, intracutaneous and intramuscular injections, drip infusions, etc. These injectable preparations may be prepared by known methods. For example, the injectable preparations may be prepared by dissolving, suspending or emulsifying the antibody of the present invention or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate dissolution aid such as an alcohol (e.g., ethanol), a polyalcohol (e.g., propylene glycol, polyethylene glycol), a nonionic surfactant (e.g., polysorbate 80, HCO-50 (polyoxyethylene (50 mols) adduct of hydrogenated castor oil)), etc. As the oily medium, there are employed, e.g., sesame oil, soybean oil, etc., which may be used in combination with a dissolution aid such as benzyl benzoate, benzyl alcohol, etc. The prepared injection is preferably filled in an appropriate ampoule. The suppository used for rectal administration may be prepared by blending the aforesaid antibody or its salt with conventional bases for suppositories.

The composition for oral administration includes a dosage form of solid or liquid, more specifically, tablets (including dragees and film-coated tablets), pills, granules, powders, capsules (including soft capsules), syrups, emulsions, suspensions, etc. Such a composition is manufactured by known methods and may contain carriers, diluents or excipients conventionally used in the field of pharmaceutical preparations. As the carriers and excipients for tablets e.g., lactose, starch, sucrose and magnesium stearate are used.

Advantageously, the pharmaceutical compositions for parenteral or oral use described above are prepared into pharmaceutical preparations with a unit dose suited to fit a dose of the active ingredients. Such unit dose preparations include, for example, tablets, pills, capsules, injections (ampoules) and suppositories. The amount of the antibody contained is generally about 5 to about 500 mg per dosage unit form; it is preferred that the aforesaid antibody is contained in about 5 to about 100 mg especially in the form of injection, and in about 10 to 250 mg for the other forms.

Each of the compositions described above may further contain other active ingredients, unless any adverse interaction occurs due to blending with the antibody described above.

The dose of the preventive/therapeutic agent or diagnostic agent (pharmaceutical) comprising the antibody of the present invention may vary depending on subject to be administered, diseases to be administered, symptoms, routes for administration, etc. When used for the treatment of, e.g., obesity in an adult patient, it is advantageous that the antibody of the present invention is intravenously administered in a single dose of normally approximately 0.01 to 20 mg/kg body weight, preferably approximately 0.1 to 10 mg/kg body weight and more preferably approximately 0.1 to 5 mg/kg body weight approximately 1 to 5 times, preferably approximately 1 to 3 times a day. For other parenteral administrations (e.g., subcutaneous administration) and oral administration, the corresponding dose may be administered. When symptoms are extremely serious, the dose may be increased depending on the conditions.

In the specification of the present invention, amino acids, etc. are shown by abbreviations and in this case, they are denoted in accordance with the IUPAC-IUB Commission on Biochemical Nomenclature or by the common codes in the art, examples of which are shown below. For amino acids that may have the optical isomer, L form is presented unless otherwise indicated.
- PAM :: phenylacetamidomethyl
- Boc: : t-butyloxycarbonyl
- Fmoc: : 9-fluorenylmethyloxycarbonyl
- Cl-Z: : 2-chloro-benzyloxycarbonyl
- Br-Z: : 2-bromo-benzyloxycarbonyl
- Bzl: : benzyl
- Cl-Bzl :: 2-chloro-benzyl
- OcHex :: cyclohexyl ester
- OBzl: : benzyl ester
- Tos: : p-toluenesulfonyl
- HONB :: N-hydroxy-5-norbornene-2,3-dicarboximido
- HOBt: :1-hydroxybenzotriazole
- HOOBt :: 3-hydroxy-3,4-dihydro-4-oxo-1,2,3-benzotriazine
- MeBzl :: 4-methylbenzyl
- Bom :: benzyloxymethyl
- Bum :: t-butoxymethyl
- Trt: : trityl
- DNP :: dinitrophenyl
- TFA: : trifluoroacetic acid
- DMF :: N,N-dimethylformamide
- DCM :: dichloromethane
- DCC :: N,N'-dichlorohexylcarbodiimide
- BHA :: benzhydrylamine
- pMBHA:: p-methylbenzhydrylamine
- CHO :: formyl
- Gly: : glycine
- Ala: : alanine
- Val: : valine
- Leu: leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagine
- Gln: glutamine

The sequence identification numbers used in the sequence listing of the specification represents the amino acid sequences of the following peptides.

### [SEQ ID NO: 1]

This shows the amino acid sequence of human urotensin II.

### [SEQ ID NO: 2]

This shows the amino acid sequence of porcine urotensin II-1.

### [SEQ ID NO: 3]

This shows the amino acid sequence of porcine urotensin II-2.

### [SEQ ID NO: 4]

This shows the amino acid sequence of bovine urotensin II.

### [SEQ ID NO: 5]

This shows the amino acid sequence of rat urotensin II.

### [SEQ ID NO: 6]

This shows the amino acid sequence of goby urotensin II.

### [SEQ ID NO: 7]

This shows the amino acid sequence of human urotensin II-related peptide (URP) obtained in REFERENCE EXAMPLE 1 described below.

### [SEQ ID NO: 8]

This shows the amino acid sequence of mouse urotensin II.

### [SEQ ID NO: 9]

This shows the amino acid sequence of the sequence of 5-10 amino acid in (the 6-11 amino acid sequence in SEQ ID NO: 2, SEQ ID NO: 3, SEQ ID NO: 4 or SEQ ID NO: 6; the 8-13 amino acid sequence in SEQ ID NO: 5; the 2-7 amino acid sequence in SEQ ID NO: 7; or the 8-13 amino acid sequence in SEQ ID NO: 8).

The hybridoma cell AUII5-6-10 obtained in EXAMPLE 1 later described has been deposited on International Patent Organisms Depository, National Institute ofAdvanced Industrial Science and Technology, located at Central 6, 1-1-1 Higashi, Tsukuba, Ibaraki (postal code: 305-8566) under Accession Number FERM BP-8221 since October 22, 2002.

The hybridoma cell AUII103-5-41 obtained in EXAMPLE 1 later described has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-8220 since October 22, 2002.

The antibody obtained from each of the hybridoma cells is shown by the cell name followed by "a."

Hereinafter, the present invention will be described in detail with reference to REFERENCE EXAMPLE and EXAMPLES, but is not deemed to limit the scope of the present invention.

### REFERENCE EXAMPLE 1

### Production of human urotensin II-related peptide (URP) (SEQ ID NO: 7)

In a reactor of peptide synthesizer ACT-90 (Advanced ChemTech, Inc.), 0.5 mmole (0.77 mmole/g resin) of Boc-Val-OCH₂-PAM resin commercially available was charged and Boc-Cys (MeBzl), Boc-Tyr(Br-Z), Boc-Lys(Cl-Z), Boc-Trp (CHO), Boc-Phe, Boc-Cys (MeBzl) and Boc-Ala in this order were introduced therein in accordance with the Boc-strategy (NMP-HOBt) peptide synthesis to give the objective protected peptide resin. After 0.32 g of this resin was distilled with 2 ml of p-cresol and 1.5 ml of 1,4-butanedithiol at 0°C for 60 minutes in 20 ml of anhydrous hydrogen fluoride, hydrogen fluoride was removed in vacuum. Diethyl ether was added to the residue and the precipitates were taken out by filtration. To the precipitates 50% aqueous acetic acid solution was added for extraction to remove insoluble matters. After the extract was sufficiently concentrated, the concentrate was applied to a Sephadex (registered trademark) G-25 column (2.0 x 80 cm) packed with 50% aqueous acetic acid solution, followed by developing with the same solvent to collect the main fractions. The fractions were lyophilized to give 118 mg of crude SH peptide. From the peptide 50 mg was taken and dissolved in 100 ml of 6M aqueous urea solution. After 400 ml of distilled water was added to dilute, pH of the dilution was adjusted to 8 with ammonia water and the mixture was gently agitated while bubbling air. The reaction was monitored on HPLC and after it was confirmed on the peaks that all the SH-form peptides were converted into the SS-form peptides, acetic acid was added to the solution to adjust the pH to 3. The solution was applied to a reversed phase chromatography column (2.6 x 60 cm) packed with LiChroprep (registered trademark) RP-18 and washed with 200 ml of 0.1% aqueous TFA and then with 200 ml of 20% acetonitrile/water containing 0.1 % TFA. Next, linear gradient elution was conducted using 300 ml of 20% acetonitrile/water containing 0.1% TFA and 300 ml of a 50% aqueous acetonitrile containing 0.1% TFA. The main fractions were collected and lyophilized to give 7.9 mg of white powders.
ESI-MS:M⁺ 1017.1 (calc. 1017.2)
Elution time on HPLC: 9.9 mins.
Column conditions
Column: Wakosil-II 5C18HG 4.6 x 100 mm
Eluent: Liner density gradient elution using Eluent A: 0.1% TFA-water and Eluent B: acetonitrile containing 0.1% TFA in A/B: 80/20~60/40 (10 mins.)
Flow rate: 1.0 ml/min.

### EXAMPLE 1

### (1) Preparation of immunogens and immunization

Using as an antigen goby (goby, long-jawed mudsucker, *Gillichthys mirabilis*) urotensin II (purchased from Peninsula Laboratories, Inc., SEQ ID NO: 6) with the C-terminal structure (Cys-Phe-Trp-Lys-Tyr-Cys) identical with human urotensin II (SEQ ID NO: 1), porcine urotensin II-1(SEQ ID NO: 2), porcine urotensin II-2 (SEQ ID NO: 3), bovine urotensin II (SEQ ID NO: 4) and rat urotensin II (SEQ ID NO: 5), antibodies recognizing the C terminus of urotensin II were prepared.

For preparation of the antigen, 1 mg of goby urotensin II peptide was bound to 4 mg of bovine thyroglobulin (BTG) using 30 mg of ECDI (1-ethyl-3-(3-dimethylaminopropyl)carbodiimide, Dojin Kagaku). Then, the reaction solution containing the resulting goby urotensin II -BTG complex was dialyzed to 0.15 M sodium chloride aqueous solution. The internal dialysate was mixed with Freund's complete adjuvant. Using the mixture as an antigen, goby urotensin II was applied to Balb/C mice (female, 6-8 weeks old) in an amount of 20 µg/animal for primary immunization. Approximately 4 weeks after the primary immunization, the complex was mixed with Freund's incomplete adjuvant and the mixture was used as an antigen for secondary immunization. The animal was boostered with a mixture of goby urotensin II -BTG complex and Freund's incomplete adjuvant every 2 other weeks until the antibody titer increased.

### (2) Preparation of biotinylated antigen

Biotin was bound to goby urotensin II to use as a labeled antigen for enzyme-linked immunoassay (EIA). That is, 2 nmols of goby urotensin II was dissolved in 0.1 ml of 50 mM phosphate buffer (pH 7.5) and 20 nmols of biotin N-hydroxysuccinimide ester was added to the solution. The mixture was reacted at room temperature for an hour. By this reaction, goby urotensin II biotinylated at the α-amino group of N-terminal alanine or goby urotensin II biotinylated at the ε-amino group of 9th lysine and goby urotensin II biotinylated at both groups were produced. The biotinylated products were fractionated on HPLC to obtain goby urotensin II biotinylated only at the N-terminal alanine residue ([N-biotinyl-Ala¹] goby urotensin II, hereinafter referred to as biotinylated goby urotensin II). The structure of goby urotensin II biotinylated only at the N-terminal alanine residue was identified by mass spectrometry to detect an increased molecular weight corresponding to one molecule of biotin bound and by N-terminal amino acid sequencing using Edman degradation, where the α-amino group of N-terminal alanine residue was biotinylated so that the reaction with phenyl isothiocyanate did not proceed and any phenylthiohydantoin derivative of the alanine residue was not detected at all.

### (3) Measurement of antibody titers

Antibody titers in mouse anti-sera during immunization of goby urotensin II were measured by the following procedures. First, in order to prepare an anti-mouse immunoglobulin antibody-bound microplate, 100 µl each of 50 mM carbonate buffer (pH 9.6) containing 10 µg/ ml of anti-mouse immunoglobulin antibody (IgG fraction, manufactured by Cappel Inc.) was dispensed in a 96-well microplate, which was allowed to stand at 4°C for 24 hours. Next, the plate was washed with phosphate buffered saline (PBS, pH 7.4). Then, 200 µl each of PBS containing 25% Block Ace (manufactured by Snow Brand Milk Products Co., Ltd.) was dispensed to block the surplus binding sites, followed by treating the plate at 4°C for at least 24 hours.

After 100 µl each of mouse anti- goby urotensin II antisera diluted with Buffer C (0.02 M phosphate buffer containing 1% BSA, 0.4 M NaCl and 2 mM EDTA, pH 7.0) was added to each well of the anti-mouse immunoglobulin antibody-bound microplate described above, the mixture was reacted at 4°C for 16 hours. The plate was then washed with PBS and 100 µl of the biotinylated goby urotensin II (diluted with Buffer C to 200-fold) prepared in (2) above was added thereto and the mixture was reacted at room temperature for 6 hours. Next, the plate was washed with PBS and 100 µl of HRP (horse radish peroxidase)-labeled avidin solution diluted with Buffer C to 10000-fold was then added to each well. The mixture was reacted at room temperature for 2 hours. After the plate was washed with PBS, 100 µl of TMB microwell peroxidase substrate system (KIRKEGAARD & PERRY LAB, INC., Funakoshi Pharmaceutical Co., Ltd.) was added thereto and the mixture was reacted at room temperature for 10 minutes thereby to assay the enzyme activity on the solid phase. After 100 µl of 1M phosphate was added to discontinue the reaction, absorbance at 450 nm was measured on a plate reader (BICHROMATIC, manufactured by Dainippon Pharmaceutical Co., Ltd.).

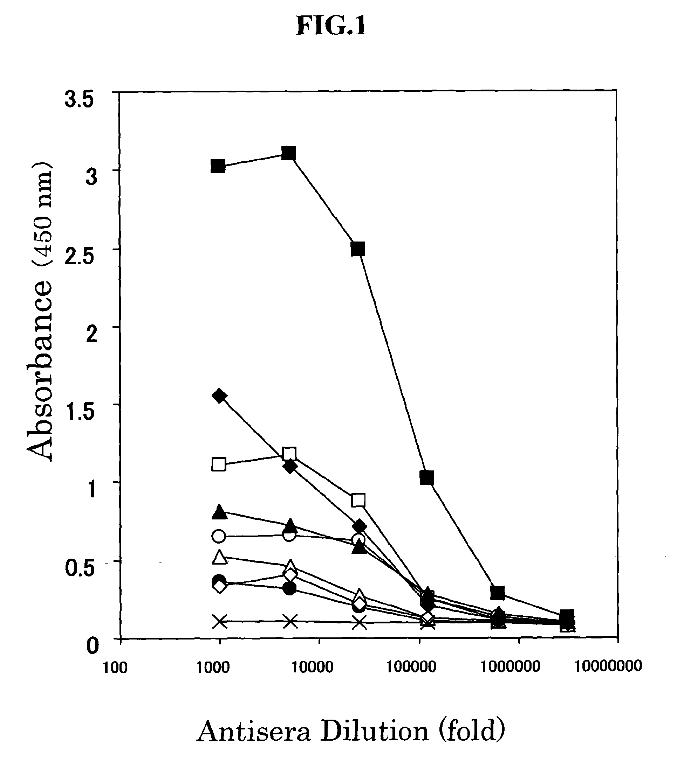

The results are shown in FIG. 1.

Increased antibody titers to the C terminus of goby urotensin II were noted in 5 out of 8 immunized mice.

### (4) Preparation of monoclonal anti-urotensin II antibodies

A solution of 200 - 300 µg of the immunogen in 0.25 - 0.3 ml of saline was intravenously injected to mouse showing a relatively high antibody titer for final immunization. The spleen was withdrawn from mouse 3 to 4 days after the final immunization, pressed against and filtered through a stainless mesh, and suspended in Eagle's minimum essential medium (MEM) to give a spleen suspension. For cell fusion, BALB/C mouse-derived myeloma cells P3-X63.Ag8.U1(P3U1) were used (Current Topics in Microbiology & Immunology, 81, 1, 1978). Cell fusion was carried out by a modification of the originally reported method (Nature, 256, 495, 1975). That is, the splenocytes and P3U1 were washed 3 times with serum-free MEM, respectively, to mix the splenocytes with P3U1 in 5:1 in terms of cell counts. The mixture was centrifuged at 800 rpm for 15 minutes to precipitate the cells. After the supernatant was thoroughly removed, the precipitates were lightly loosened and 0.3 ml of 45% polyethylene glycol (PEG) 6000 (manufactured by Kochleit) was added thereto. The mixture was settled at 37°C for 7 minutes in a thermostat for cell fusion. After completion of the fusion, MEM was added to the cells at a rate of 2 ml/min. to reach 15 ml of MEM in total. The mixture was then centrifuged at 600 rpm for 15 minutes to remove the supernatant. The cell deposits were suspended in GIT medium (Wako Pure Chemical Industries, Ltd.) (GIT-10% FCS) supplemented with 10% fetal calf serum in 2 x 10⁵ cells/ml, and the suspension was plated onto 120 wells of a 24-well Multidish (manufactured by Linbro Chemical Co.) in 1 ml each/well. After plating, the cells were incubated at 37°C in a 5% CO₂ incubator. Twenty-four hours after, GIT-10% FCS medium containing HAT (1 x 10⁻⁴ M hypoxanthine, 4 x 10⁻⁷ M aminopterin and 1.6 x 10⁻³ M thymidine) (HAT medium) was added to the wells in 1 ml each/well to initiate HAT selection culture. After 1 ml of the old medium was discarded on Days 3, 6 and 9 subsequent to the culture initiation, HAT selection culture was continued by supplementing 1 ml of HAT medium. Hybridomas were found to grow on Days 9 to 14 after the cell fusion. When the culture medium turned yellow (about 1 x 10⁶ cells/ml), the supernatant was collected and the antibody titers were assayed in accordance with the procedures described in (3) above. In order to confirm binding specificity of the antibody in the hybridoma supernatant to biotinylated goby urotensin II, it was examined at this point of time if the binding would be inhibited with 1 µM porcine urotensin II-1.

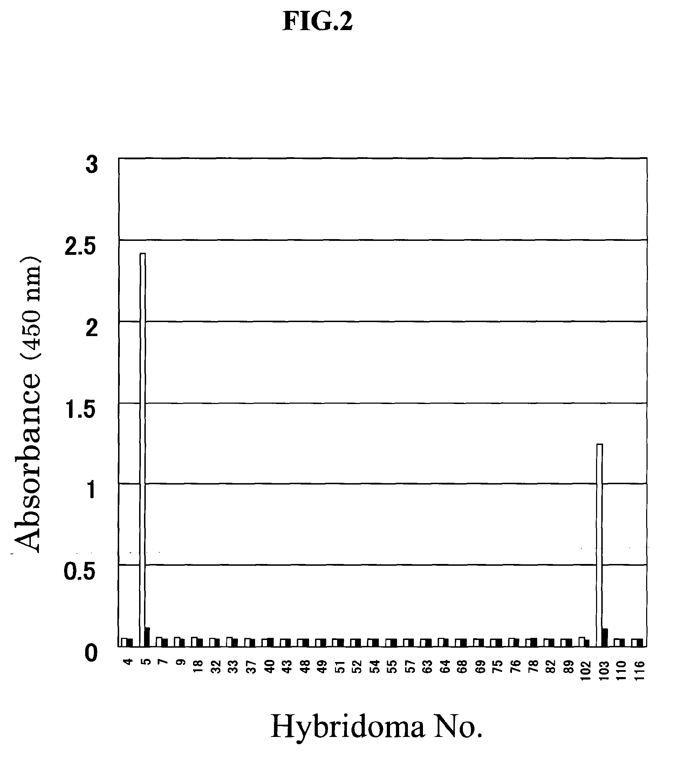

As a typical example of screening of mouse-derived hybridoma immunized with goby urotensin II, the results obtained using mouse No. 6 (see FIG. 1) are shown in FIG. 2.

The antibodies in the hybridoma supernatants from No. 5 and No. 103 were found to specifically bind to urotensin II. Thus, total 2 hybridomas from No. 5 and No. 103 were selected.

Next, these hybridomas were cloned by limiting dilution. In cloning, thymocytes from BALB/C mice were added as feeder cells to the wells in 5 x 10⁵ cells/well. After the cloning, 2 clones of No. 5-6-10 and No. 103-5-41 were selected as hybridomas showing a higher antibody production level. Hybridomas No. 5-6-10 and No. 103-5-41 were named AUII5-6-10 and AUII103-5-41, respectively.

After cloning, each hybridoma was intraperitoneally given in a dose of 1 to 3x10⁶ cells/animal to mice (BALB/C), to which 0.5 ml of mineral oil had previously been given intraperitoneally, and the ascites containing the antibodies were collected 6 to 20 days after.

The monoclonal antibodies were purified from the collected ascites through Protein A column. That is, 6 to 20 ml of the ascites was diluted with an equal amount of a binding buffer (1.5 M glycine containing 3.5 M NaCl and 0.05% NaN₃, pH 9.0). The dilution was then provided onto Recombinant Protein A-Agarose (manufactured by Repligen Corp.), which had been previously equilibrated with the binding buffer to elute the specific antibody with an eluting buffer (0.1 M citrate buffer containing 0.05%NaN₃, pH 3.0). The eluate was dialyzed to PBS at 4°C for 2 days, then subjected to bacteria-free filtration through 0.22 µm filter (manufactured by Millipore Inc.) and stored at 4°C or -80°C. To identify the class/subclass of the monoclonal antibody, enzyme-linked immunosorbent assay (ELISA) using a purified monoclonal antibody-bound solid phase was carried out. That is, 100 µl each of 0.1 M carbonate buffer solution, pH 9.6, containing 2 µg/ ml of the antibody was dispensed to a 96-well microplate, which was allowed to stand at 4°C for 24 hours. Following the procedures described in (3) above, the surplus binding sites in the wells were blocked with Block Ace. Thereafter, the class/subclass of the immobilized antibodies was identified by ELISA using an isotype typing kit (Mouse-Typer™ Sub-Isotyping Kit, manufactured by Biorad Inc.). The classes of the antibodies produced from the two hybridomas (No. 5-6-10 and No. 103-5-41) were both found to belong to IgG1.

### EXAMPLE 2

### Enzyme immunoassayby competitive method

The reaction specificity of the monoclonal antibodies (AUII5-6-10a and AUII103-5-41a) produced by the respective two hybridomas No.5-6-10 and No.103-5-41, which were prepared using goby urotensin II as an immunogen, was examined by the following procedures.

To the anti-mouse immunoglobulin antibody-bound microplate described in EXAMPLE 1 (3) above, 33 µl of a 486-fold dilution of the AUII5-6-10 hybridoma culture supernatant diluted with Buffer C (0.02 M phosphate buffer containing 1% BSA, 0.4 M NaCl and 2 mM EDTA, pH 7.0) or 33 µl of a 54-fold dilution of the AUII103-5-41 hybridoma culture supernatant diluted with Buffer C, 33 µl each of human, porcine-1, bovine, rat and goby urotensin II solutions in various concentrations prepared using Buffer C, and 33 µl of the biotinylated goby urotensin II (diluted with Buffer C to 8333-fold) were added, and each mixture was reacted at 4°C for 16 hours. After completion of the reaction, the mixture was washed with PBS and 100 µl of HRP-labeled avidin solution diluted with Buffer C to 10000-fold was added to each well, followed by reacting at room temperature for 3 hours. After completion of the reaction, the mixture was washed with PBS and the enzyme activity on the solid phase was assayed by the method described in EXAMPLE 1 (3) above.

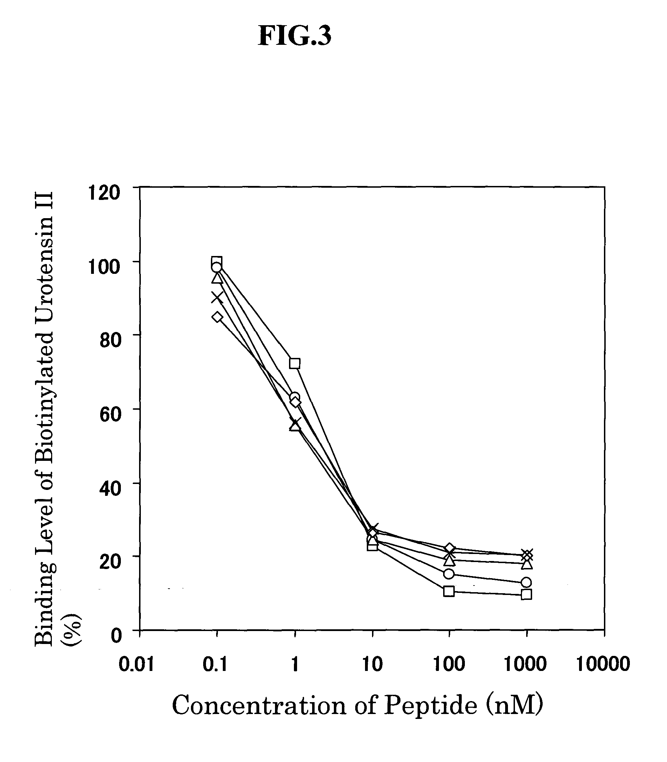

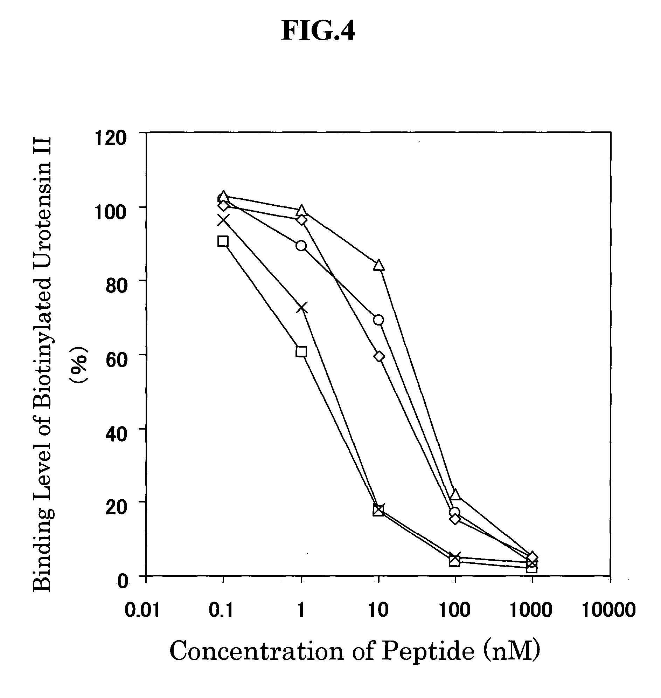

The results of competitive EIA obtained using the culture supernatants of these hybridomas (AUII5-6-10 and AUII103-5-41) are shown in FIGS. 3 and 4.

As shown in FIG. 3, it was noted that AUII5-6-10a displayed a similar reactivity to any of human, porcine-1, bovine, rat and goby urotensin II peptides. It is considered based on these results that AUII5-6-10a recognizes the Cys-Phe-Trp-Lys-Tyr-Cys sequence, which is a partial structure commonly possessed by these peptides. From the binding inhibition curve of AUII5-6-10a for human urotensin II, the human urotensin II level for (B/B₀)=0.5 was found to be 1.2 nM.

On the other hand, as shown in FIG. 4, AUII103-5-41a displayed a higher reactivity with human and goby urotensin II, as compared to the reactivity with porcine-1, bovine and rat urotensin II. The reactivity of AUII103-5-41a with human urotensin II (the antigen level which gives (B/ B₀) = 0.5: 0.68 nM) was about 0.04 time the reactivity with porcine urotensin II-1(the antigen level which gives (B/B₀) = 0.5: 17.4 nM). From this it is considered that AUII103-5-41a will recognize the Asp-Cys-Phe-Trp-Lys-Tyr-Cys sequence or the partial structure commonly possessed in human and goby urotensin II.

The level of human urotensin II, which gives (B/ B₀) = 0.5 in these two hybridoma culture supernatants, is in a range of 0.5 to 2.0 nM, indicating that the competitive-EIA using these two hybridoma culture supernatants is highly sensitive. Thus, about 0.2 nM [(B/ B₀) = 0.9] human urotensin II could be detected.

### EXAMPLE 3

### Neutralizing actions on the biological activities of human, porcine-1, bovine, rat and goby urotensin II with monoclonal antibody AUII5-6-10a

The neutralizing actions on the biological activities of human, porcine-1, bovine, rat and goby urotensin II with monoclonal antibody AUII5-6-10a were determined by the assay system for arachidonate metabolite releasing activity using rat GPR14 receptor expression CHO cells (the same cells as the rat SENR expression CHO cells described in WO 00/32627).

AUII5-6-10a was diluted to various concentrations (1, 3, 10, 30, 100 and 300 nM) and the dilution was incubated with human, porcine-1, bovine, rat and goby urotensin II (10 nM each) at room temperature for an hour. The residual activity was then assayed using rat GPR14 receptor expression CHO cells.

The arachidonate metabolite releasing activity was assayed as follows. Rat GPR14 receptor expression CHO cells were plated on a 24-well plate at a cell density of 0.5 x 10⁵ cells/well. After incubation for 24 hours, [³H] arachidonic acid was added to the wells in 0.5µCi/well. Twenty-four hours after the addition of [³H] arachidonic acid, the cells were washed with MEM containing 0.1 % BSA and a solution mixture of the monoclonal antibody in each concentration described above with human, porcine-1, bovine, rat and goby urotensin II was added thereto in 500 µl/well. After incubation at 37°C for an hour, 400 µl out of 500 µl of the reaction solution was added to 4 ml of a scintillator to monitor the amount of [³H] arachidonate metabolite released into the reaction solution on a scintillation counter.

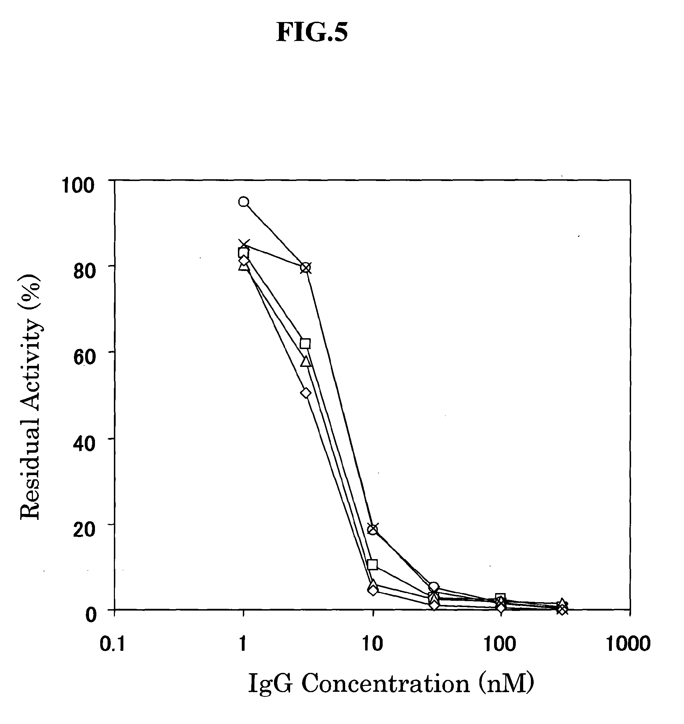

The results are shown in FIG. 5.

AUII5-6-10a suppressed 100% of the activities of human, porcine-1, bovine, rat and goby urotensin II by 3-fold or 10-fold amount (in a molar ratio).

The foregoing results reveal that AUII5-6-10a neutralizes the arachidonate metabolite releasing activities of human, porcine-1, bovine, rat and goby urotensin II.

### EXAMPLE 4

### Anti-anxiety-like action of AUII5-6-10a in the hole-board test system

ICR (CD-1) mice (9-19 weeks old weighing 36-38 g, male, Charles River Japan, Inc.) were mildly anesthetized with diethyl ether and mouse IgG (immunoglobulin G) (Sigma, 10 mg/ ml PBS, 5µl) or AUII5-6-10a (10mg/ml PBS, 5 µl) was given to the right ventricle. A double needle (Matsumoto Seisakusho) was used for intraventricular administration. Thirty minutes after the mice awaken were placed in a restraint cage (Natsume Seisakusho Co., Ltd.) and exposed to restraint stress for an hour. Control animal was allowed to freely behave in a breeding cage for an hour. Spontaneous locomotor activity and peeping behavior were quantified for subsequent 5 minutes. For the measurement, the spontaneous motor activity monitoring system (Muromachi Kikai Co., Ltd.) was employed. The peeping behavior of mice was quantified with an apparatus consisting of a rearing sensor (MRX-110TX-RX) equipped at the lower side of the cage and a board with holes therethrough (3.8 cm in diameter, 4 holes) being suspended from the upper part by 5 mm above the sensor to fix inside the cage. The number of peeping was expressed in terms of the intercepting count of the mounted sensor with mice placed on the board by poking the head out from the holes due to its peeping behavior. At the same time, total spontaneous motor activity was measured with a SUPERMEX sensor (PYS-001, a passive infrared sensor) mounted to the upper part. The total spontaneous motor activity was expressed by the number that mice crossed the sensor. Experiment was carried out between 15:00 and 18:00.

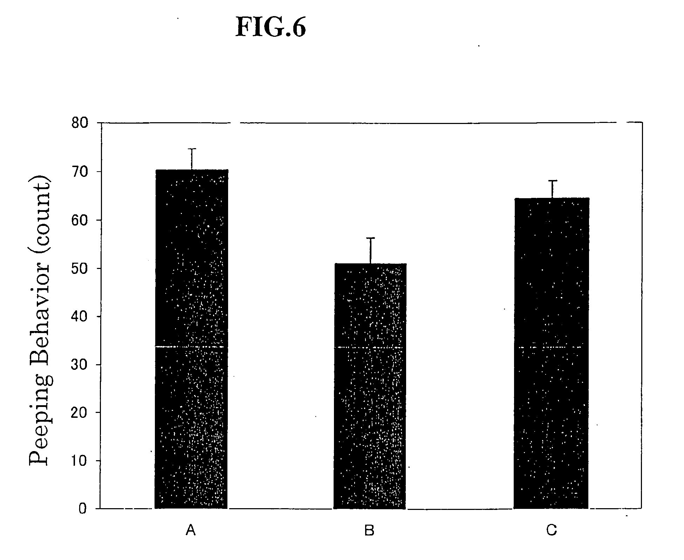

The results are shown below (FIG. 6).
Administered with mouse IgG (non-stressed): 70.5 ± 4.2 counts (n=21)
Administered with mouse IgG (exposed to restraint stress): 51.1 ± 5.3 counts (n=19), p<0.01
Administered with AUII5-6-10a (exposed to restraint stress): 64.6 ± 3.6 counts (n=21), p<0.05

These results reveal that by exposure to restraint stress for an hour, the peeping behavior of mice was significantly reduced and further that intraventricular administration of AUII5-6-10a significantly recovered the peeping behavior reduced by the restraint stress.

In this case, no change in the total spontaneous motor activity of mice was observed [administered with mouse IgG (non-stressed): 621.5 ± 20.4 counts; administered with mouse IgG (exposed to restraint stress): 611.2 ± 29.6 counts; administered with AUII5-6-10a (exposed to restraint stress): 649.7 ± 20.1 counts]

The foregoing results reveal that intraventricular administration of AUII5-6-10a suppressed anxiety-like behavior shown by the reduced peeping behavior of mice induced by restraint stress, without affecting any spontaneous behavior.

### INDUSTRIAL APPLICABILITY

The antibody of the present invention is useful for development of therapeutic agents, preventive agents and diagnostic agents for diseases associated with the peptide of the present invention. By using hybridoma cells containing the antibody of the present invention, the antibody of the present invention can be manufactured in an industrial scale. In addition, the pharmaceuticals (especially diagnostic agents) comprising the antibody of the present invention are useful for diagnosis of diseases associated with the peptide of the present invention [for example, central nerve diseases (e.g., Alzheimer's disease, Parkinsonian syndrome, Pick's disease, Huntington's disease, senile dementia, cerebrovascular dementia, etc.), mental disorders (e.g., anxiety, depression, insomnia, schizophrenia, phobia, etc.), circulatory diseases (e.g., hypertension, hypotension, etc.), heart diseases (e.g., heart failure, arrhythmia, long QT syndrome, dilated congestive cardiomyopathy, hypertrophic cardiomyopathy, pulmonary hypertension, etc.), renal diseases (e.g., nephritis, renal failure, interstitial renal disorders, etc.), urinary tract disorders (e.g., pollakiuria, urinary incontinence, etc.), or the like].

The antibody of the present invention has the activity of neutralizing the peptide of the present invention and hence, is useful as the preventive/therapeutic agent for central nerve diseases (e.g., Alzheimer's disease, Parkinsonian syndrome, Pick's disease, Huntington's disease, senile dementia, cerebrovascular dementia, etc.), mental disorders (e.g., anxiety, depression, insomnia, schizophrenia, phobia, etc.), circulatory diseases (e.g., hypertension, hypotension, etc.), heart diseases (e.g., heart failure, arrhythmia, long QT syndrome, dilated congestive cardiomyopathy, hypertrophic cardiomyopathy, pulmonary hypertension, etc.), renal diseases (e.g., nephritis, renal failure, interstitial renal disorders, etc.), urinary tract disorders (e.g., pollakiuria, urinary incontinence, etc.), or the like.

By using the antibody of the present invention, the amount of the peptide of the present invention can be measured with a high sensitivity. Therefore, the quantifying method of the present invention is useful for diagnosis, prevention or treatment for the diseases associated with the peptide of the present invention [for example, central nerve diseases (e.g., Alzheimer's disease, Parkinsonian syndrome, Pick's disease, Huntington's disease, senile dementia, cerebrovascular dementia, etc.), mental disorders (e.g., anxiety, depression, insomnia, schizopluenia, phobia, etc.), circulatory diseases (e.g., hypertension, hypotension, etc.), heart diseases (e.g., heart failure, arrhythmia, long QT syndrome, dilated congestive cardiomyopathy, hypertrophic cardiomyopathy, pulmonary hypertension, etc.), renal diseases (e.g., nephritis, renal failure, interstitial renal disorders, etc.), urinary tract disorders (e.g., pollakiuria, urinary incontinence, etc.), or the like]. By using the antibody of the present invention, the amount of the peptide of the present invention derived from human, swine, bovine, rat, mouse, goby, etc. can be determined with a high sensitivity and is thus useful as the diagnostic agent, preventive/therapeutic agent, reagent, etc. for diseases associated with the peptide of the present invention.

## Claims

1. An antibody reacting specifically reacting with a partial peptide in the C-terminal region of a polypeptide having the amino acid sequence represented by SEQ ID NO: 1, SEQ ID NO: 2, SEQ ID NO: 3, SEQ ID NO: 4, SEQ ID NO: 5, SEQ ID NO: 6 or SEQ ID NO: 8, or a derivative of said polypeptide.

2. The antibody according to claim 1, which specifically reacts with a partial peptide in the C-terminal region of a polypeptide having the amino acid sequence represented by SEQ ID NO: 1, SEQ ID NO: 2, SEQ ID NO: 3, SEQ ID NO: 4, SEQ ID NO: 5 or SEQ ID NO: 6, or a derivative of said polypeptide.

3. The antibody according to claim 1, which specifically reacts with a peptide having the amino acid sequence represented by SEQ ID NO: 9.

4. The antibody according to claim 1, wherein the partial peptide in the C-terminal region is a peptide having (i) the 5-10 amino acid sequence in SEQ ID NO: 1, (ii) the 6-11 amino acid sequence in SEQ ID NO: 2, SEQ ID NO: 3, SEQ ID NO: 4 or SEQ ID NO: 6, (iii) the 8-13 amino acid sequence in SEQ ID NO: 5, (iv) the 2-7 amino acid sequence in SEQ ID NO: 7, or (v) the 8-13 amino acid sequence in SEQ ID NO: 8.

5. The antibody according to claim 1, which is a monoclonal antibody.

6. The antibody according to claim 1, which is labeled.

7. The antibody according to claim 1, which is a neutralizing antibody.

8. The antibody according to claim 7, which neutralizes the activity of a polypeptide having the amino acid sequence represented by SEQ ID NO: 1, SEQ ID NO: 2, SEQ ID NO: 4, SEQ ID NO: 5, SEQ ID NO: 6, SEQ ID NO: 7 or SEQ ID NO: 8, or a derivative thereof.

9. The antibody according to claim 7, which neutralizes the activity of a polypeptide having the amino acid sequence represented by SEQ ID NO: 1, SEQ ID NO: 2, SEQ ID NO: 4, SEQ ID NO: 5 or SEQ ID NO: 6.

10. The antibody according to claim 5, which is shown by AUII5-6-10a capable of being produced from a hybridoma cell shown by AUII5-6-10 (FERM BP-8221).

11. The antibody according to claim 5, which is shown by AUII103-5-41a capable of being produced from a hybridoma cell shown by AUIII03-5-41 (FERM BP-8220).

12. A hybridoma cell capable of producing the antibody according to claim 5.

13. The hybridoma cell according to claim 12, which is shown by AUII103-5-41 (FERM BP-8220).

14. The hybridoma cell according to claim 12, which is shown by AUII5-6-10 (FERM BP-8221).

15. A method of producing the antibody according to claim 5, which comprises culturing the hybridoma cell according to claim 12 in vivo or in vitro and collecting the antibody according to claim 5 from the body fluid or its culture.

16. A pharmaceutical comprising the antibody according to claim 1.

17. The pharmaceutical according to claim 16, which is a preventive/therapeutic agent for central nerve diseases, mental disorders, circulatory diseases, heart diseases, renal diseases or urinary tract disorders.

18. A diagnostic agent comprising the antibody according to claim 1.

19. The diagnostic agent according to claim 18, which is a diagnostic agent for central nerve diseases, mental disorders, circulatory diseases, heart diseases, renal diseases or urinary tract disorders.

20. A method of quantifying a polypeptide having the amino acid sequence represented by SEQ ID NO: 1, SEQ ID NO: 2, SEQ ID NO: 3, SEQ ID NO: 4, SEQ ID NO: 5, SEQ ID NO: 6 or SEQ ID NO: 8, or a derivative thereof, which comprises using the antibody according to claim 1.

21. A method of quantifying a polypeptide having the amino acid sequence represented by SEQ ID NO: 1, SEQ ID NO: 2, SEQ ID NO: 3, SEQ ID NO: 4, SEQ ID NO: 5, SEQ ID NO: 6 or SEQ ID NO: 8, or a derivative thereof, in a test fluid, which comprises competitively reacting the antibody according to claim 1, a test fluid and a labeled form of polypeptide having the amino acid sequence represented by SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8, or a derivative of said polypeptide, and determining a ratio of the labeled polypeptide having the amino acid sequence represented by SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8, or a derivative thereof, bound to the antibody.

22. A method for diagnosis of a disease associated with a polypeptide having the amino acid sequence represented by SEQ ID NO: 1, SEQ ID NO: 2, SEQ ID NO: 3, SEQ ID NO: 4, SEQ ID NO: 5, SEQ ID NO: 6, SEQ ID NO: 7 or SEQ ID NO: 8, or a derivative thereof, which comprises using the antibody according to claim 1.

23. A method of preventing/treating central nerve diseases, mental disorders, circulatory diseases, heart diseases, renal diseases or urinary tract disorders, which comprises administering an effective dose of the antibody according to claim 1 to a mammal.

24. Use of the antibody according to claim 1 for manufacturing a preventive/therapeutic agent for central nerve diseases, mental disorders, circulatory diseases, heart diseases, renal diseases or urinary tract disorders.
